# FASCICULE DE BREVET EUROPEEN

(11) **EP 1 795 897 B1**
(45) Date de publication et mention de la délivrance du brevet: **18.02.2009**
(21) Numéro de dépôt: 06125912.3
(22) Date de dépôt: 12.12.2006
(51) Int. Cl.: G01N 33/50, G01N 33/574, C12N 5/06

(54) **Test prédictif de réponse thérapeutique du cancer du sein**
Prädiktiver Test auf das Ansprechen auf eine Brustkrebstherapie
Predictive test of therapeutic response of breast cancer

(30) Priorité: 12.12.2005 FR 0512555
(43) Date de publication de la demande: 13.06.2007
(73) Titulaire: Université de Limoges, 87000 Limoges (FR)
(72) Inventeur: Lautrette, Christophe, 87000 Limoges (FR)
(74) Mandataire: Ahner, Francis

(56) Documents cités:
- WO-A-97/19189
- LU-A1- 86 721
- US-A- 5 328 844
- US-A- 5 736 129
- TAETLE R ET AL: "ROLE OF TRANSFERRIN IRON AND TRANSFERRIN RECEPTORS IN MYELOID LEUKEMIA CELL GROWTH STUDIES WITH AN ANTITRANSFERRIN RECEPTOR MONOCLONAL ANTIBODY" JOURNAL OF CLINICAL INVESTIGATION, vol. 75, no. 3, 1985, pages 1061-1067, XP002389142 ISSN: 0021-9738
- SINCLAIR J ET AL: "EFFECTS OF INSULIN AND INSULIN-LIKE GROWTH FACTOR I ON GROWTH OF HUMAN LEUKEMIA CELLS IN SERUM-FREE AND PROTEIN-FREE MEDIUM" BLOOD, vol. 72, no. 1, 1988, pages 66-72, XP002389143 ISSN: 0006-4971
- JAYME D W ET AL: "CULTURE MEDIA FOR PROPAGATION OF MAMMALIAN CELLS, VIRUSES, AND OTHER BIOLOGICALS" ADVANCES IN BIOTECHNOLOGICAL PROCESSES, ALAN R. LISS, NEW YORK, NY, US, vol. 5, 1985, pages 1-30, XP009023800 ISSN: 0736-2293
- DARBRE P ET AL.: "Effects of Estradiol and Tamoxifen on Human Breast Cancer Cells in Serum-free Culture" CANCER RESEARCH, vol. 44, 1984, pages 2790-2793,

## Description

La présente invention concerne une nouvelle méthode pour tester des médicaments ou candidats médicament *ex-vivo* sur culture cellulaire de tumeurs du sein comprenant un milieu de culture permettant la conservation et la croissance des cellules tumorales au détriment des cellules stromales et ceci pour toute cellule de cancers du sein quelque soit le patient. Cette méthode permet de déterminer une chimiothérapie optimale et spécifique avec un ou plusieurs médicaments pour chaque patient en un temps d'une semaine environ.

### INTRODUCTION

Le traitement des patients atteints de cancer a traditionnellement été basé sur la sélection d'agents actifs et sur des combinaisons identifiées par expérimentations cliniques randomisées de phases III.

L'identification de nouveaux agents de chimiothérapie, et la validation clinique de leur activité spécifique pour un cancer donné par des données statistiques significatives est, en oncologie, la clé de l'émergence de nouveaux traitements de référence, appelés protocoles de consensus. De plus, l'impact de ces nouvelles approches thérapeutiques sur la survie des patients peut être comparé avec celle des protocoles précédents.
L'évaluation de l'utilité clinique des traitements développés grâce à cette approche a notamment permis d'augmenter de façon significative la survie des femmes atteintes d'un cancer ovarien ou du sein a des stades avancés.
Pourtant, malgré l'apparition de nouveaux médicaments et de nouvelles stratégies thérapeutiques, la progression du nombre de cancer et de la mortalité des patients constituent un problème majeur du cancer du sein, intrinsèque à la tumeur ou acquis, qui résulte très largement des mécanismes de résistance aux médicaments.

Ainsi, alors que les protocoles de chimiothérapies actuels pour le cancer du sein entraînent une réponse clinique favorable dans seulement 60 % des cas, la récidive à 5 ans est en outre à 50 %.

Afin d'améliorer les réponses thérapeutiques et la survie des patients, les recherches se sont orientées vers la mise au point de tests biologiques destinés à déterminer l'activité potentielle d'une molécule au niveau des cellules tumorales d'un malade avant traitement. Ce concept a été appliqué au cancer, afin de prédire l'efficacité de molécules chez des malades à partir de culture de fragment de leur tumeur. Ces essais, réalisés au début des années 1990, n'ont pas permis d'obtenir des résultats suffisamment efficients pour justifier la systématisation des tests *ex-vivo* de chimiosensibilité.

On sait aujourd'hui que l'échec de cette approche est dû à la présence dans la tumeur des cellules non tumorales provenant du stroma, qui venait biaiser les résultats de l'analyse. Ainsi, l'objectif aujourd'hui est d'obtenir des résultats exploitables grâce à des systèmes de culture pouvant limiter la pousse des cellules stromales et permettre le développement en culture des cellules tumorales.

Dans le cadre de la présente invention, nous avons développé un système complet pour permettre d'anticiper l'efficacité de différentes molécules sur la tumeur d'un malade à partir d'un prélèvement de sa tumeur en moins de 10 jours. Contrairement aux autres approches, nous bloquons la croissance des cellules du stroma grâce à un milieu de culture spécifique qui permet en outre une forte prolifération des cellules tumorales. De plus, nos milieux biologiques sont asériques et donc chimiquement définis ce qui permet une reproductibilité maximale des analyses.

DARBRE P ET AL.: "Effects of Estradiol and Tamoxifen on Human Breast Cancer Cells in Serum-free Culture" CANCER RESEARCH, vol. 44, 1984, pages 2790-2793 divulgent un milieu qui sert à la croissance d'une lignée de cellules tumorales mammaires comprenant
- 0,01 mg/L d'hydrocortisone;
- 0,00065 mg/L de Tri-iodo-thyronine;
- 3 mg/L d'insuline;
- 5 mg/L de transférine;
- 0,005 mg/L d'EGF humain recombinant ; et
- 0,0002 72 mg/L de 1,7-Estradiol.

### DESCRIPTION

Ainsi, dans un premier aspect, l'invention concerne une méthode pour tester *ex-vivo* ou *in-vitro* l'efficacité de médicaments ou candidats médicament pour le traitement des cancers du sein, ci-après dénommée "oncogramme", comprenant :
a) la mise en culture de cellules de cancer du sein provenant d'un échantillon dans un milieu de base de cellules tumorales caractérisé en ce qu'il comprend la combinaison des facteurs de croissance et des ingrédients suivants :

| | |
|---|---|
| Hydrocortisone | entre 0,5 et 0,3 mg/L |
| Tri-iodo-thyronine | entre 0,0008 et 0,0004 mg/L |
| Insuline | entre 15 et 5 mg/L |
| Transferine | entre 10 et 4 mg/L |
| EGF humain recombinant | entre 0,15 et 0,05 mg/L |
| et 17β-Estradiol | entre 0,3 et 0,2 mg/L |

jusqu'à l'obtention d'une culture cellulaire de cellules tumorales essentiellement dépourvue de cellules stromales,
b) la mise en contact de la culture cellulaire obtenue à l'étape a) avec un ou plusieurs médicaments ou candidats médicament;
c) la détermination et la sélection du ou des médicaments ou candidats médicament présentant une activité anti-proliférative.

Par activité anti-proliférative, on entend une diminution d'au moins 50%, de préférence d'au moins 75% ou encore 90% du nombre de cellules tumorales dans le milieu de culture.

Avantageusement, les cellules de cancer du sein sont mises en culture à l'étape a) dans un milieu tel que défini ci-dessous :

| | |
|---|---|
| Hydrocortisone | 0,4 mg/L |
| Tri-iodo-thyronine | 0,0006 mg/L |
| Insuline | 10 mg/L |
| Transferine | 5,5 mg/L |
| EGF humain recombinant | 0,1 mg/L |
| 17β-Estradiol | 0,25 mg/L |

Avantageusement, le milieu ci-dessus comprend également :
- Albumine de sérum bovin entre 1200 et 500 mg/L, de préférence 1000 mg/L, et/ou
- Sodium Sélénite entre 0,01 et 0,007 mg/L, de préférence 0,004 mg/L.

Par milieu de culture de base de cellules tumorales, on peut citer plus particulièrement le milieu MBT suivant :

### Milieu MBT : Milieu Basal de Tumeur

| COMPOSANTS | Concentration (mg/L) |
|---|---|
| Glycine | 26.25 |
| L-Alanine | 13.35 |
| L-Arginine hydrochloride | 147.5 |
| L-Asparagine-H2O | 20.7 |
| acide L-Aspartic | 19.95 |
| L-Cysteine hydrochloride-H2O | 17.56 |
| L-Cystine 2HCl | 45.99 |
| L-Glutamic Acid | 7.35 |
| L-Glutamine | 365 |
| L-Histidine hydrochloride-H2O | 31.48 |
| L-Isoleucine | 54.47 |
| L-Leucine | 59.05 |
| L-Lysine hydrochloride | 91.25 |
| L-Methionine | 17.24 |
| L-Phenylalanine | 35.48 |
| L-Proline | 28.75 |
| L-Serine | 36.75 |
| L-Threonine | 53.45 |
| L-Tryptophan | 9.02 |
| L-Tyrosine disodium salt dihydrate | 55.79 |
| L-Valine | 52.85 |
| Biotine | 0.0035 |
| Choline chloride | 8.98 |
| D-Calcium pantothenate | 2.24 |
| Acide Folic | 2.65 |
| i-Inositol | 12.6 |
| Niacinamide | 2.02 |
| Pyridoxine hydrochloride | 2 |
| Riboflavin | 0.219 |
| Thiamine hydrochloride | 2.17 |
| Vitamin B12 | 0.68 |
| Chlorure de Calcium (CaCl2) (anhydre) | 116.6 |
| Cupric sulfate (CuSO4-5H2O) | 0.0013 |
| Ferric Nitrate (Fe(NO3)3-9H2O) | 0.05 |
| Ferric sulfate (FeSO4-7H2O) | 0.417 |
| Chlorure de Magnésium (anhydre) | 28.64 |
| Sulfate de Magnesium (MgSO4) (anhydre) | 48.84 |
| Chlorure de Potassium (KCl) | 311.8 |
| Bicarbonate de Sodium (NaHCO3) | 1200 |
| Chlorure de Sodium (NaCl) | 6995.5 |
| Phosphate de Sodium dibasic (Na2HPO4) anhydre | 71.02 |
| Phosphate de Sodium monobasic (NaH2PO4-H2O) | 62.5 |
| Sulfate de Zinc (ZnSO4-7H2O) | 0.432 |
| D-Glucose (Dextrose) | 3151 |
| HEPES | 3574.5 |
| Hypoxanthine Na | 2.39 |
| Linoleic Acid | 0.042 |
| Lipoic Acid | 0.105 |
| Putrescine 2HCl | 0.081 |
| Pyruvate de Sodium | 55 |
| Thymidine | 0.365 |

Dans un mode de réalisation particulier, la méthode décrite ci-dessus comprend en outre le transport de l'échantillon avant la mise en culture dans un milieu du type des milieux de base de cellules tumorales, de la pénicilline (100 U/ml) et de la streptomycine (100 µg/ml). Ce milieu permet d'éviter la contamination de l'échantillon pendant son acheminement jusqu'au site où les tests sont effectués.
Avantageusement, on rajoute de l'albumine (par exemple à 1mg/ml) ce qui permet de maintenir un taux de survie des cellules pendant le transport supérieur à 90% environ.

La méthode décrite ci-dessus peut également comprendre une étape de dissociation de l'échantillon tissulaire au moyen d'une solution isotonique ou d'un milieu comprenant de la collagénase de type II (1,5 mg/ml) et de la trypsine (0,5%) pendant environ 3 heures, par exemple de 2 heures à 4 heures. Cette étape permet d'accélérer la dissociation cellulaire tout en maintenant une viabilité optimale des cellules.

De préférence, le test de la population cellulaire à l'étape c) est réalisé après environ 5 jours de culture. En effet, à ce stade la méthode de culture selon l'invention permet d'obtenir des cultures de cellules tumorales dépourvues de cellules issues du stroma. A cet effet, une mise en oeuvre visée est l'utilisation de Bromo-desoxy-Uridine (BrdU) comme marqueur de cellules en prolifération. L'invention vise également toute alternative connue de l'homme du métier pour déterminer le nombre de cellules vivantes dans le milieu ou la mortalité cellulaire, telle que la coloration au bleu Trypan.

Dans un autre aspect, l'invention porte sur un milieu de culture tel que défini ci-avant, en particulier un milieu de culture du type des milieux de culture de cellules tumorales et comprenant la combinaison des facteurs de croissance et des ingrédients suivants :

| | |
|---|---|
| Hydrocortisone | entre 0,5 et 0,3 mg/L |
| Tri-iodo-thyronine | entre 0,0008 et 0,0004 mg/L |
| Insuline | entre 15 et 5 mg/L |
| Transferine | entre 10 et 4 mg/L |
| EGF humain recombinant | entre 0,15 et 0,05 mg/L |
| et 17β-Estradiol | entre 0,3 et 0,2 mg/L |

Avantageusement, ce milieu comporte en outre :
- Albumine de sérum bovin entre 1200 et 500 mg/L, de préférence 1000 mg/L, et/ou
- Sodium Sélénite entre 0,01 et 0,004 mg/L, de préférence 0.007 mg/L.

L'invention porte également sur une culture cellulaire comprenant le milieu cité ci-dessus et des cellules de cancer du sein.

L'invention porte également sur un kit pour la mise en oeuvre de la méthode selon l'invention, dénommé oncogramme, caractérisé en ce qu'il comprend un milieu de culture, un milieu de transport et un milieu de dissociation, les dits milieux étant définis ci-dessus.

Dans un troisième aspect l'invention porte sur l'utilisation d'une culture cellulaire décrite précédemment pour tester *ex-vivo* l'efficacité de médicaments ou candidats médicament en vue d'établir un traitement sur-mesure du cancer du sein adapté à chaque patient. L'invention vise également l'utilisation de la dite culture cellulaire pour le criblage de molécules susceptibles d'exercer une activité anti-proliférative.

### Exemple 1 : Transport de l'échantillon

1.1 Après exérèse ou biopsie d'une tumeur du sein, une fraction du tissu tumoral frais est prélevée (de quelques millimètres à plusieurs centimètres selon la taille de la tumeur et les besoins pour les autres analyses).
Cette fraction de tumeur est alors mise dans un milieu de conservation et gardée à + 4° C pendant 24 ou 48 heures.

Différents milieux ont été testés afin d'obtenir un taux maximal de survie des cellules tumorales. Ces milieux sont :
- Tampon phosphate salin (PBS)
- Milieu MBT (Milieu Basal de Tumeur, composition en annexe 1)
- Milieu MBT supplémenté avec de l'albumine (1 mg/ml).
- Milieu MBT supplémenté avec de la pénicilline (100 U/ml) et de la streptomycine (100 µg/ml).
- Milieu MBT supplémenté avec de l'albumine (1 mg/ml), de la pénicilline (100 U/ml), et de la streptomycine (100 µg/ml).

Nous avons testé différents milieux permettant de conserver l'échantillon tumoral pendant 24 heures, et leur efficacité a été analysée selon 2 critères : la viabilité globale des cellules au moment de leur mise en culture, et d'autre part l'absence de contamination détectable au microscope après la mise en culture des cellules.

| Milieux de conservation testés : | Viabilité cellulaire après 24 de conservation des tissus tumoraux dans le milieu de transport (exprimé en % de cellules viables) |
|---|---|
| 1 : PBS | 83 (± 6) |
| 2 : Milieu MBT | 93 (± 4) |
| 3 : Milieu MBT + albumine (1 mg/ml) | 95 (± 4) |
| 4 : Milieu MBT + pénicilline (100 U/ml) + streptomycine (100 µg/ml) | 84 (± 7) |
| 5 : Milieu MBT + pénicilline (100 U/ml) + streptomycine (100 µg/ml) + albumine (1 mg/ml) | 93 (± 5) |

La viabilité globale des cellules lors de la mise en culture après conservation dans les milieux 1 et 4 est inférieure à celle des milieux 2, 3, et 5.

De plus, les milieux de conservation 1, 2 et 3 sont associés dans nos conditions de culture des tumeurs à des contaminations (levures et/ou bactérie) observables sous microscope alors qu'aucune contamination n'a été observée avec une utilisation des milieux de conservation 4 et 5.

Nous avons également comparé la viabilité cellulaire au moment de la mise en culture des cellules après 24 heures et 48 heures dans le milieu 5. Aucune différence n'a été observée entre 24 et 48 heures de conservation à + 4°C du fragment de tumeur dans le milieu 5 (respectivement 94 ± 4% *vs* 93 ± 1% de cellules viables, nombre d'échantillons testés = 10).
Le milieu 5 permet à l'échantillon d'être conservé à + 4°C jusqu'à 48 heures sans altérer significativement la viabilité des cellules et sans contamination microbiologique du prélèvement.

### Exemple 2 : Préparation de l'échantillon

Les cellules de l'échantillon de tumeur sont ensuite dissociées selon des méthodes optimisées tant physiques que chimiques.
L'échantillon est dans un premier temps lavé 4 fois 1 minute dans un milieu MBT contenant de la pénicilline (1000 U/ml) et de la streptomycine (1000 µg/ml) puis rincé 1 minute dans le milieu MBT.
Il est ensuite découpé mécaniquement en fragments cubiques de 1 mm.
Les fragments sont alors repris dans le milieu de dissociation. Plusieurs protocoles ont été testés afin d'obtenir une séparation unicellulaire des fragments de tumeurs :
- Protocole de dissociation 1 : collagénase de type II (1,5 mg/ml) pendant 2 heures 30 minutes puis trypsine (0,5%) pendant 30 minutes
- Protocole de dissociation 2 : trypsine (0,5%) pendant 30 minutes puis collagénase de type II (1,5 mg/ml) pendant 2 heures 30 minutes
- Protocole de dissociation 3 : collagénase de type II (1,5 mg/ml) et trypsine (0,5%) pendant 3 heures
Les fragments ont toujours été incubés à 37°C sous agitation légère dans une étuve à 5% de CO₂.
La suspension cellulaire est ensuite filtrée au travers d'un tamis cellulaire de 40 µm et la trypsine inactivée grâce à une solution d'inhibiteur de trypsine extraite du soja (0,5 mg / ml en concentration finale).
La viabilité cellulaire est alors vérifiée par une coloration au bleu Trypan et une numération à la cellule de Malassez.
L'objectif de la préparation de l'échantillon était d'obtenir avec une incubation de 3 heures maximum une dissociation totale des fragments de la tumeur tout en conservant une viabilité cellulaire proche de 90% au moment de la mise en culture des cellules.

Nos travaux ont montré que les protocoles 1 et 2 ne permettaient qu'une dissociation partielle des fragments de tumeur après 3 heures d'incubation (observation sous microscope). Seul le protocole de dissociation 3 [collagénase de type II (1,5 mg/ml) et trypsine (0,5%)] permet d'obtenir en 3 heures une dissociation complète du fragment de tissu tumoral.
De plus, ce protocole permet d'obtenir une viabilité cellulaire moyenne à la mise en culture de 93 ± 3 % (nombre d'échantillons testés = 10).

### Exemple 3 : Culture des cellules tumorales du malade

La suspension cellulaire est alors centrifugée pendant 12 minute à 300g.
Le culot cellulaire est repris dans le milieu de l'invention décrit ci-dessus.
Les cellules sont ensuite ensemencées dans des plaques pour culture cellulaire à raison de 10⁵ cellules par ml pendant 5 jours. Le milieu est renouvelé après 3 jours de culture.
Après 5 jours de culture, la viabilité cellulaire est de nouveau vérifiée par une coloration au bleu Trypan et une numération à la cellule de Malassez.

### Exemple 4 : Caractérisation des cellules en culture

### 4.1 Recherche de critères tumoraux

Les cellules cultivées ont été caractérisées par marquage histologique trichromique Hemalun Eosine Safran au laboratoire d'anatomopathologie du CHRU de Limoges suivant le protocole standard utilisé pour l'identification et la classification des tumeurs. La caractérisation cytologique des cellules tumorales de nos cultures a été réalisée par un médecin-biologiste du CHRU de Limoges.

La caractérisation cytologique des cellules dans nos cultures a permis d'identifier un grand nombre de cellules présentant un ou plusieurs critères tumoraux définis selon la classification standard utilisée en anatomopathologie.

### 4.2 Etude du lignage cellulaire

Le lignage cellulaire des cellules en culture a également été vérifié par marquage en immunofluorescence indirecte.
Après un lavage en PBS, les cellules sont fixées avec une solution de paraformaldéhyde dilué à 4 % en PBS pendant 30 minutes à température ambiante (TA) puis lavées 3 fois en PBS. Les cellules sont alors fixées rapidement par séchage sur une lame en verre pour immunofluorescence à 16 puits. Les cellules sont perméabilisées par une solution à 90 % d'éthanol glacial pendant 1 minute et lavées 3 fois en PBS. Une étape de saturation visant à bloquer les sites de fixation non spécifique est réalisée par l'incubation des cellules avec une solution saturante de sérum d'agneau normal dilué à 10 % dans du PBS pendant 30 minutes à TA.
Les cellules sont ensuite incubées avec l'anticorps primaire : des anticorps de souris anti-cytokératines humaines (AE1 / AE3) dilué en PBS-sérum d'agneau à 10% au 1/50 pendant 1 heure à TA. Comme contrôle négatif, des cellules sont incubées dans les mêmes conditions sans anticorps primaire.

Après 3 lavages en PBS, les cellules sont incubées avec l'anticorps secondaire de chèvre anti- immunoglobulines totales de souris couplé à de l'Alexa Fluor 488 dilué au 1/10 000 en PBS pendant 30 minutes à TA.

Après 3 rinçages dans du PBS, une contre-coloration des cellules au bleu Evans est alors réalisée. Après 3 lavages dans du PBS et un rinçage rapide à l'eau, les cellules sont montées entre lame et lamelle dans une solution de glycérol-gélatine (Gibco) et observées au microscope à fluorescence (Zeiss) ou en microscopie confocale (Zeiss, LSM510).

Les tumeurs du sein sont d'origine épithéliale. L'anticorps monoclonal AE3/AE5, qui reconnaît spécifiquement les cellules épithéliales du tissu mammaire et ne marque pas les cellules du stroma, a été utilisé, en immunofluorescence indirecte, comme marqueur des cellules tumorales en culture. Une contre-coloration au bleu Evans a été réalisée pour rechercher la présence dans les cultures de cellules d'origine non-épithéliale (cellules issues du stroma).

Un marquage homogène intracellulaire observé avec l'anticorps AE3/AE5 permet donc d'identifier les cellules tumorales présentes dans les cultures. La contre-coloration au bleu Evans permet de détecter de cellules non tumorales.

Nous avons réalisé cette étude sur des cellules de tumeur avant leur mise en culture (après la dissociation cellulaire), et nous avons comparé les résultats avec ceux obtenus après 5 jours de culture des cellules de ces tumeurs.

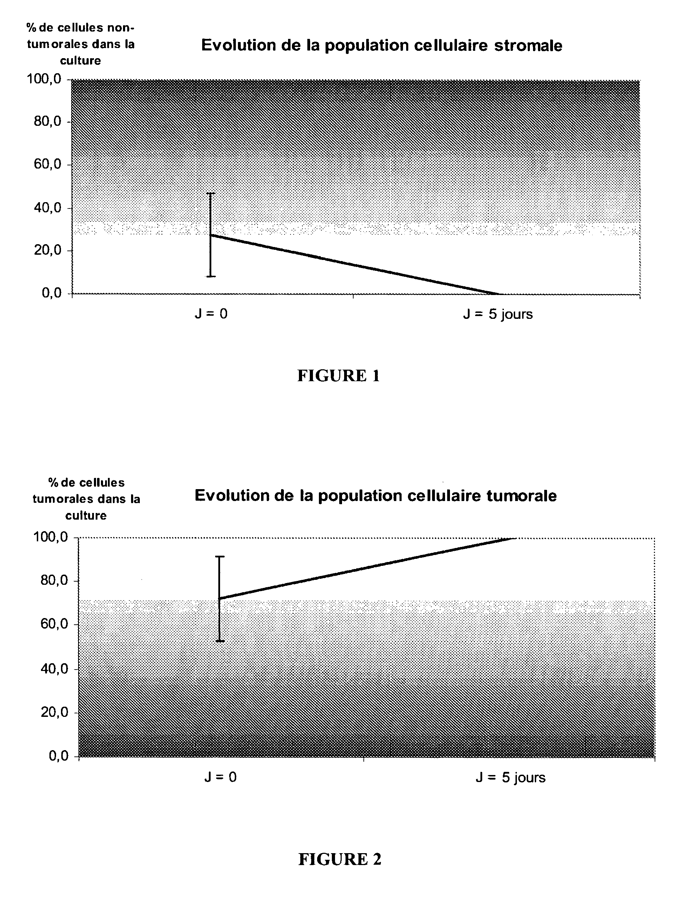

Nous avons observé, après 5 jours de culture dans nos conditions, une disparition de la population cellulaire stromale (**Figure 1**). De plus, après 5 jours de culture dans nos conditions, on observe un enrichissement de la culture cellulaire en cellules tumorales (**Figure 2**).

Nous avons étudié la viabilité cellulaire après 5 jours de culture par une coloration au bleu Trypan et observé une viabilité cellulaire moyenne de 97 ± 1% (nombre d'échantillons testés = 10).

### 4.3 Analyse de la prolifération cellulaire

Les cellules en prolifération après 5 jours de culture ont été identifiées par marquage de l'ADN des cellules en division au Bromo-desoxy-Uridine (BrdU).
La prolifération des cellules a également été étudiée avec des concentration en EGF de 0,02 et 0,05 mg /ml pour vérifier son impact après 5 jours de culture sur le taux de prolifération des cellules tumorales.
Après 5 jours de culture, les cellules ont été exposées 48 ou 72 heures au BrdU (20 µM).
Après un lavage en PBS, les cellules sont alors traitées comme précédemment en remplaçant l'anticorps primaire par un anticorps anti- BrdU (clone BU-33) utilisé au 1/100.

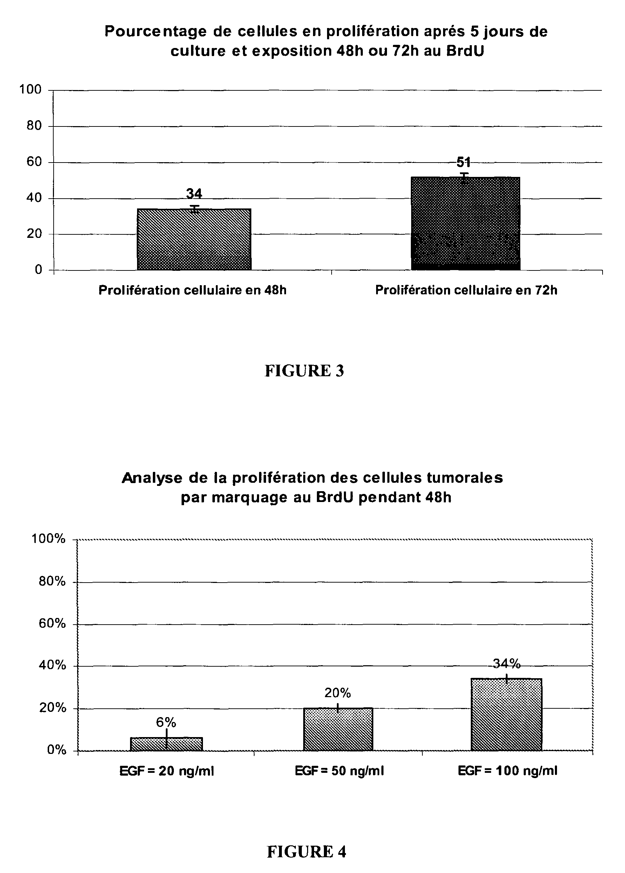

Le nombre de cellules ayant incorporé du BrdU dans leur ADN a été compté sous microscope (comptage de 100 cellules au minimum dans 5 champs au moins) et comparé au nombre total de cellules dans ces champs (cellules colorées au bleu Evans).
Après 5 jours de culture, les cellules en division ont été identifiées par l'incorporation de BrdU pendant 48 heures ou 72 heures.
Un tiers des cellules en culture sont en prolifération après 7 jours de culture, et près de la moitié sont en prolifération après 8 jours de culture (**Figure 3**).
Nous avons ensuite étudié le rôle de la concentration en EGF sur la prolifération des cellules en culture.
Des écarts de prolifération cellulaire sont observés entre des concentrations de 20, 50, ou 100 ng/ml. Afin d'augmenter dans les cultures le nombre de cellules issues du fragment de tumeur d'un malade, une concentration de 100 ng/ml d' EGF permet d'obtenir une meilleure prolifération cellulaire (**Figure 4**).

### Conclusion

L'oncogramme est un procédé global qui prend en compte à la fois des critères scientifiques (méthodes de dissociation et de culture de cellules tumorales) mais également des nécessités médicales (transport de l'échantillon tumoral depuis une clinique éloignée, forte expansion des cellules pour les échantillons de très petite taille, temps total nécessaire à l'analyse restreint).
Ainsi, l'échantillon tumoral doit pourvoir être conservé jusqu'à 48 heures à + 4°C sans altérer significativement la viabilité des cellules. Ce délai correspond au temps nécessaire aux sociétés spécialisées pour acheminer en France les échantillons à un laboratoire d'analyse.
Lors de nos essais, nous avons montré que le milieu MBT permettait de conserver à + 4°C l'échantillon de tumeur avec une viabilité cellulaire à la mise en culture élevée (93%), et de plus supérieure à celle d'un simple milieu physiologique (83% pour le tampon phosphate salin). De plus, l'adjonction de pénicilline et de streptomycine dans le milieu de conservation permettait d'éviter les contaminations microbiennes des cultures de tumeur.

En effet, les fragments sont issus des laboratoires d'anatomo-pathologie et sont donc récupérés dans des conditions non stériles. Notre protocole est adapté à l'élimination des contaminations qui pourraient se développer pendant le transport limitant ainsi les échecs de mise en cultures des cellules tumorales de malades et réduisant aussi les manipulations ultérieures destinées à « décontaminer » le fragment tumoral.
Globalement, le milieu n°5 [cf annexe 2, Milieu de Conservation de Tumeur (MCT)] permet à la fois de conserver à + 4°C l'échantillon de tumeur de 24 à 48 heures sans altérer la viabilité des cellules, mais également d'éviter la contamination microbienne des cellules tumorales en culture.
L'échantillon tumoral doit ensuite être traité pour obtenir une culture de cellules dissociées. Les tumeurs du sein sont des tissus très compacts, dont la résistance est proche de celle du cartilage. Pour cela, nous avons mis au point un protocole et des milieux de dissociation qui permettent de séparer très rapidement les cellules, tout en conservant la viabilité cellulaire.
Le protocole de dissociation 3, associant conjointement la collagénase de type II et la trypsine, est le seul permettant d'obtenir en 3 heures une dissociation cellulaire complète de l'échantillon tout en conservant une viabilité cellulaire moyenne de 93 %. En effet, des protocoles utilisant des concentrations supérieures de trypsine et de collagénase permettent de réduire ce temps d'incubation, mais aux dépens de la viabilité cellulaire globale. De même, les données de la littérature rapportent fréquemment des conditions d'incubation du fragment de tumeur pendant 12 heures à + 4°C avec de la trypsine. Cependant, ce protocole entraîne une forte augmentation du temps global du procédé, et de plus risque d'entraîner une diminution de la viabilité des cellules lors de leur mise en culture. Pour cela, le procédé de dissociation de l'échantillon tumoral avec le milieu de dissociation 3 est spécifiquement adapté au cahier des charges de l'oncogramme.
Les cellules séparées sont alors mises en culture dans un milieu spécifiquement défini pour, d'une part, favoriser la multiplication des cellules tumorales et d'autre part empêcher celle des cellules du stroma. Cette approche permet de séparer les cellules tumorales des cellules normales qui les entourent et constituent le stroma ce qui permet d'analyser par la suite uniquement les cellules tumorales. En effet, les travaux précédents d'autres équipes ont clairement montrés que les cellules non tumorales du stroma entraînaient des biais lors des analyses *ex-vivo* de tumeurs de malades.
L'analyse de la population cellulaire, réalisée après 5 jours de culture, a permis de montrer que notre protocole permet d'obtenir des cultures de cellules tumorales dépourvues de cellules issues du stroma. Le milieu de culture complet (composé du milieu MBT présenté en annexe 1 auquel est ajouté des facteurs de complément présentés en annexe 3) permet donc de discriminer, dans un système de culture cellulaire, les cellules tumorale et les cellules stroma.
De plus, contrairement à un milieu de culture conventionnel contenant un pourcentage variable de sérum de veau foetal décomplémenté, le milieu de culture utilisé est entièrement défini, c'est à dire que chacun de ses constituants est connu ainsi que sa concentration. Ceci assure une reproductibilité technique, ainsi qu'une capacité d'adaptation car le rôle de chacun des composants est connu et peut être adapté selon les nécessités de l'expérience. En outre, des facteurs de croissance sont introduits dans le milieu car ils sont connus pour favoriser l'émergence clinique chez les malades de métastases tumorales. Le milieu de culture défini va donc permettre de sélectionner parmi les cellules tumorales en culture les populations à potentiel métastatique. Cette sélection est importante car ces cellules sont les plus agressives, au niveau médical, pour le malade, et elles doivent être ciblées en priorité par les agents thérapeutiques.
Nous avons également étudié la prolifération cellulaires après 5 jours de culture, durant 48 heures ou 72 heures. Nos protocoles de culture permettent d'obtenir une forte prolifération cellulaire, avec 34 % des cellules en division à 48 heures, et environ la moitié des cellules en division à 72 heures. Cette forte prolifération des cellules tumorales permet d'augmenter de manière importante la population cellulaire, et de réaliser des analyses de prolifération et de mortalité des cellules tumorales après 5 jours de culture, alors que les cellules sont en phase de croissance.

Ainsi, notre protocole permet d'étudier l'effet de différents facteurs sur les cellules tumorales d'un malade. Après 5 jours de culture, les cellules tumorales sont exposées au(x) facteur(s) dont on souhaite déterminer les effets durant 48 heures. Des cellules non exposées servent de référence.

Un point également important du procédé est son universalité puisque notre approche nous a permis de réaliser des cultures de cellules tumorales à partir de plus de 20 tumeurs du sein issues de différents malades.

Les applications du procédé sont cliniques, lorsque les facteurs sont ceux utilisés lors du traitement du cancer. Toutefois, des applications pharmaceutiques peuvent également être réalisées lorsque les facteurs sont des candidat-médicaments encore en phase de sélection et qui nécessitent des validations chez l'homme.

Il est ainsi possible d'étudier l'activité métabolique des cellules par l'étude de la dégradation du MTT par les déshydrogénases mitochondriales des cellules vivantes. La viabilité est estimée selon l'intensité colorimétrique de la réaction grâce à un spectrophotomètre. Les viabilités cellulaires sans et avec les facteurs sont alors comparées.

D'autres techniques peuvent être employées comme le marquage des cellules en apoptose par TUNEL, ou par l'utilisation d'Annexine V et de iodure de propidium ; la recherche de marqueur par immuno-marquage ou par Western-blotting ; ou encore des études moléculaires d'expression de gène.

Ainsi, le procédé et les milieux de l'invention (oncogramme) permettent d'obtenir en 5 jours, à partir d'un fragment de tumeur du sein d'un malade prélevé dans les 48 heures, un modèle d'étude *ex-vivo* des cellules tumorale du malade.

Couplé à des techniques classiques d'analyses biologiques, l'oncogramme présente des applications importantes à la fois cliniques et pharmaceutiques, pour anticiper l'efficacité d'un facteur sur les cellules tumorales d'un malade, et ainsi personnaliser la prise en charge thérapeutique du cancer du sein.

## Revendications

1. Méthode pour tester ex-vivo l'efficacité de médicaments ou candidats médicament pour le traitement des cancers du sein **caractérisée en ce qu'**elle comprend :
a) la mise en culture de cellules de cancer provenant d'un échantillon du sein dans un milieu de base de cellules tumorales comprenant la combinaison des facteurs de croissance et des ingrédients suivants :
| | |
|---|---|
| Hydrocortisone | entre 0,5 et 0,3 mg/L |
| Tri-iodo-thyronine | entre 0,0008 et 0,0004 mg/L |
| Insuline | entre 15 et 5 mg/L |
| Transferine | entre 10 et 4 mg/L |
| EGF humain recombinant | entre 0,15 et 0,05 mg/L |
| et 17β-Estradiol | entre 0,3 et 0,2 mg/L |
jusqu'à l'obtention d'une culture cellulaire de cellules tumorales essentiellement dépourvue de cellules stromales,
b) la mise en contact de la culture cellulaire obtenue à l'étape a) avec un ou plusieurs médicaments ou candidats médicament;
c) la détermination et la sélection du ou des médicaments ou candidats médicament présentant une activité anti-proliférative.

2. Méthode selon la revendication 1, **caractérisée en ce que** le milieu de l'étape a) comprend :
| | |
|---|---|
| Hydrocortisone | 0,4 mg/L |
| Tri-iodo-thyronine | 0,0006 mg/L |
| Insuline | 10 mg/L |
| Transferine | 5,5 mg/L |
| EGF humain recombinant | 0,1 mg/L |
| 17β-Estradiol | 0,25 mg/L |

3. Méthode selon l'une des revendications 1 et 2, **caractérisée en ce que** le milieu de l'étape a) comprend en outre :
- Albumine de sérum bovin entre 1200 et 500 mg/L, de préférence 1000 mg/L, et/ou
- Sodium Sélénite entre 0,01 et 0,004 mg/L, de préférence 0.007 mg/L.

4. Méthode selon l'une des revendications 1 à 3, **caractérisée en ce qu'**elle comprend en outre le transport de l'échantillon avant la mise en culture dans un milieu du type des milieux de base de cellules tumorales comprenant de la pénicilline, notamment à 100 U/ml, et de la streptomycine, notamment à 100 µg/ml.

5. Méthode selon la revendication 5, **caractérisée en ce que** le milieu de transport comprend en outre de l'albumine, notamment à 1 mg/ml.

6. Méthode selon l'une des revendications 1 à 5, **caractérisée en ce qu'**elle comprend en outre une étape de dissociation de l'échantillon tissulaire au moyen d'une solution isotonique ou d'un milieu comprenant de la collagénase de type II, notamment à 1,5 mg/ml, et de la trypsine, notamment à 0,5%, l'échantillon tissulaire étant traité pendant environ 3 heures.

7. Méthode selon l'une des revendications 1 à 6, **caractérisée en ce que** l'étape c) est réalisée après environ 5 jours de culture.

8. Méthode selon l'une des revendications 1 à 7, **caractérisée en ce que** l'étape c) comprend la détermination du nombre de cellules vivantes dans le milieu ou un test de mortalité cellulaire, notamment une coloration au bleu Trypan ou l'utilisation de Bromo-desoxy-Uridine (BrdU) comme marqueur de cellules en prolifération.

9. Milieu de culture pour cellules de cancer du sein du type des milieux de culture de cellules tumorales, **caractérisé en ce qu'**il comprend la combinaison des facteurs de croissance et des ingrédients suivants :
| | |
|---|---|
| Hydrocortisone | entre 0,5 et 0,3 mg/L |
| Tri-iodo-thyronine | entre 0,0008 et 0,0004 mg/L |
| Insuline | entre 15 et 5 mg/L |
| Transferine | entre 10 et 4 mg/L |
| EGF humain recombinant | entre 0,15 et 0,05 mg/L |
| et 17β-Estradiol | entre 0,3 et 0,2 mg/L |

10. Milieu de culture selon la revendication 9 comprenant en outre :
- Albumine de sérum bovin entre 1200 et 500 mg/L, de préférence 1000 mg/L, et/ou
- Sodium Sélénite entre 0,01 et 0,004 mg/L, de préférence 0.007 mg/L.

11. Culture cellulaire comprenant le milieu selon la revendication 9 ou 10 et des cellules de cancer du sein.

12. Kit pour la mise en oeuvre de la méthode selon l'une des revendications 1 à 8 **caractérisé en ce qu'**il comprend un milieu de culture selon la revendication 9 ou 10, un milieu de transport défini à la revendication 4 ou 5 et un milieu de dissociation défini à la revendication 6.

13. Utilisation d'une culture cellulaire selon la revendication 11 pour tester ex-vivo l'efficacité de médicaments ou candidats médicaments en vue d'établir un traitement surmesure du cancer du sein adapté à chaque patient.

14. Utilisation d'une culture cellulaire selon la revendication 11 pour le criblage de molécules susceptibles d'exercer une activité anti-proliférative.

## Claims

1. A method for *ex-vivo* testing of the effectiveness of drugs or drug candidates for treating breast cancers, **characterized in that** said method comprises:
a) culturing cancer cells from a breast tissue sample in a basal medium for tumor cells comprising the following combination of growth factors and ingredients:
| | |
|---|---|
| Hydrocortisone | between 0.5 and 0.3 mg/l |
| Triiodothyronine | between 0.0008 and 0.0004 mg/l |
| Insulin | between 15 and 5 mg/l |
| Transferrin | between 10 and 4 mg/l |
| Recombinant human EGF | between 0.15 and 0.05 mg/l |
| and 17β-Estradiol | between 0.3 and 0.2 mg/l |
until a tumor cell culture essentially free of stromal cells is obtained;
b) placing the cell culture obtained in step a) in contact with one or more drugs or drug candidates;
c) determining and selecting the drugs or drug candidates that exhibit anti-proliferative activity.

2. The method of claim 1, **characterized in that** the medium of step a) comprises:
| | |
|---|---|
| Hydrocortisone | 0.4 mg/l |
| Triiodothyronine | 0.0006 mg/l |
| Insulin | 10 mg/l |
| Transferrin | 5.5 mg/l |
| Recombinant human EGF | 0.1 mg/l |
| 17β-Estradiol | 0.25 mg/l |

3. The method of one of claims 1 and 2, **characterized in that** the medium of step a) further comprises:
- bovine serum albumin between 1200 and 500 mg/l, preferably 1000 mg/l, and / or
- sodium selenite between 0.01 and 0.004 mg/l, preferably 0.007 mg/l.

4. The method of one of claims 1 to 3, **characterized in that** said method further comprises the transport of the sample before culturing in a basal medium for tumor cells comprising penicillin, notably at 100 U/ml, and streptomycin, notably at 100 µg/ml.

5. The method of claim 4, **characterized in that** the transport medium further comprises albumin, notably at 1 mg/ml.

6. The method of one of claims 1 to 5, **characterized in that** said method further comprises a step of dissociating the tissue sample by means of an isotonic solution or a medium comprising type II collagenase, notably at 1.5 mg/ml, and trypsin, notably at 0.5%, the tissue sample being treated for approximately 3 hours.

7. The method of one of claims 1 to 6, **characterized in that** step c) is carried out after approximately 5 days of culture.

8. The method of one of claims 1 to 7, **characterized in that** step c) includes determining the number of living cells in the medium or a cell mortality test, notably staining with trypan blue or using bromodeoxyuridine (BrdU) as a marker of proliferating cells.

9. A culture medium for breast cancer cells of the type used as culture media for tumor cells, **characterized in that** said culture medium for breast cancer cells comprises the following combination of growth promoters and ingredients:
| | |
|---|---|
| Hydrocortisone | between 0.5 and 0.3 mg/l |
| Triiodothyronine | between 0.0008 and 0.0004 mg/l |
| Insulin | between 15 and 5 mg/l |
| Transferrin | between 10 and 4 mg/l |
| Recombinant human EGF | between 0.15 and 0.05 mg/l |
| and 17β-Estradiol | between 0.3 and 0.2 mg/l |

10. The culture medium of claim 9, **characterized in that** said medium further comprises:
- bovine serum albumin between 1200 and 500 mg/l, preferably 1000 mg/l, and / or
- sodium selenite between 0.01 and 0.004 mg/l, preferably 0.007 mg/l.

11. A cell culture comprising the medium according to claim 9 or 10 and breast cancer cells.

12. A kit for implementing the method of one of claims 1 to 8, **characterized in that** said kit comprises the culture medium of claim 9 or 10, the transport medium defined in claim 4 or 5 and the dissociation medium defined in claim 6.

13. Use of the cell culture of claim 11 for ex-*vivo* testing of the effectiveness of drugs or drug candidates with a view to establishing a breast cancer treatment specifically tailored for each patient.

14. Use of the cell culture of claim 11 to screen molecules likely to exhibit anti-proliferative activity.

## Patentansprüche

1. Verfahren, um ex *vivo* die Wirksamkeit von Arzneimitteln oder Arzneimittelkandidaten für die Behandlung von Krebserkrankungen der Brust zu testen, **dadurch gekennzeichnet, dass** es umfasst:
a) das Inkulturnehmen von Krebszellen, die von einer Probe der Brust stammen, in einem Grundmedium für Tumorzellen, welches die Kombination der folgenden Wachstumsfaktoren und Bestandteile umfasst:
| | |
|---|---|
| Hydrocortison | zwischen 0,5 und 0,3 mg/l |
| Triiodthyronin | zwischen 0,0008 und 0,0004 mg/l |
| Insulin | zwischen 15 und 5 mg/l |
| Transferrin | zwischen 10 und 4 mg/l |
| humaner rekombinanter EGF | zwischen 0,15 und 0,05 mg/l |
| und 17β-Estradiol | zwischen 0,3 und 0,2 mg/l, |
bis zur Erzielung einer Zellkultur von Tumorzellen, welche im Wesentlichen frei von Stromazellen ist,
b) das Inkontaktbringen der in dem Schritt a) erhaltenen Zellkultur mit einem oder mehreren Arzneimitteln oder Arzneimittelkandidaten;
c) das Bestimmen und die Selektion des oder der Arzneimittel oder Arzneimittelkandidaten, das bzw. der bzw. die eine antiproliferative Aktivität aufweist bzw. aufweisen.

2. Verfahren nach Anspruch 1, **dadurch gekennzeichnet, dass** das Medium des Schritts a) umfasst:
| | |
|---|---|
| Hydrocortison | 0,4 mg/l |
| Triiodthyronin | 0,0006 mg/l |
| Insulin | 10 mg/l |
| Transferrin | 5,5 mg/l |
| humanen rekombinanten EGF | 0,1 mg/l |
| 17β-Estradiol | 0,25 mg/l. |

3. Verfahren nach einem der Ansprüche 1 und 2, **dadurch gekennzeichnet, dass** das Medium des Schritts a) außerdem umfasst:
- Rinderserumalbumin zwischen 1200 und 500 mg/l, vorzugsweise 1000 mg/l und/oder
- Natriumselenit zwischen 0,01 und 0,004 mg/l, vorzugsweise 0,007 mg/l.

4. Verfahren nach einem der Ansprüche 1 bis 3, **dadurch gekennzeichnet, dass** es außerdem das Transportieren der Probe vor dem Inkulturnehmen in einem Medium vom Typ der Grundmedien für Tumorzellen, welches Penicillin, insbesondere in einer Konzentration von 100 E/ml, und Streptomycin, insbesondere in einer Konzentration von 100 µg/ml, umfasst.

5. Verfahren nach Anspruch 5, **dadurch gekennzeichnet, dass** das Transportmedium außerdem Albumin, insbesondere in einer Konzentration von 1 mg/ml, umfasst.

6. Verfahren nach einem der Ansprüche 1 bis 5, **dadurch gekennzeichnet, dass** es außerdem einen Schritt einer Dissoziation der Gewebeprobe mittels einer isotonischen Lösung oder eines Mediums, welches Collagenase vom Typ II, insbesondere in einer Konzentration von 1,5 mg/ml, und Trypsin, insbesondere in einer Konzentration von 0,5%, umfasst, umfasst, wobei die Gewebeprobe während etwa 3 Stunden behandelt wird.

7. Verfahren nach einem der Ansprüche 1 bis 6, **dadurch gekennzeichnet, dass** der Schritt c) nach etwa 5 Tagen Kultur ausgeführt wird.

8. Verfahren nach einem der Ansprüche 1 bis 7, **dadurch gekennzeichnet, dass** der Schritt c) die Bestimmung der Anzahl von lebenden Zellen in dem Medium oder einen Zellmortalitätstest, insbesondere eine Färbung mit Trypanblau oder die Verwendung von Bromdesoxyuridin (BrdU) als Marker von in Proliferation befindlichen Zellen, umfasst.

9. Kulturmedium für Brustkrebszellen vom Typ der Kulturmedien für Tumorzellen, **dadurch gekennzeichnet, dass** es die Kombination der folgenden Wachstumsfaktoren und Bestandteile umfasst:
| | |
|---|---|
| Hydrocortison | zwischen 0,5 und 0,3 mg/l |
| Triiodthyronin | zwischen 0,0008 und 0,0004 mg/l |
| Insulin | zwischen 15 und 5 mg/l |
| Transferrin | zwischen 10 und 4 mg/l |
| humaner rekombinanter EGF | zwischen 0,15 und 0,05 mg/l |
| und 17β-Estradiol | zwischen 0,3 und 0,2 mg/l. |

10. Kulturmedium nach Anspruch 9, welches außerdem umfasst:
- Rinderserumalbumin zwischen 1200 und 500 mg/l, vorzugsweise 1000 mg/l und/oder
- Natriumselenit zwischen 0,01 und 0,004 mg/l, vorzugsweise 0,007 mg/l.

11. Zellkultur, welche das Medium nach Anspruch 9 oder 10 und Brustkrebszellen umfasst.

12. Kit für das Ausführen des Verfahrens nach einem der Ansprüche 1 bis 8, **dadurch gekennzeichnet, dass** er ein Kulturmedium nach Anspruch 9 oder 10, ein in Anspruch 4 oder 5 definiertes Transportmedium und ein in Anspruch 6 definiertes Dissoziationsmedium umfasst.

13. Verwendung einer Zellkultur nach Anspruch 11, um ex *vivo* die Wirksamkeit von Arzneimitteln oder Arzneimittelkandidaten zu testen in Hinblick darauf, eine Behandlung von Brustkrebs nach Maß, die an jeden Patienten angepasst ist, festzulegen.

14. Verwendung einer Zellkultur nach Anspruch 11 für das Screening auf Moleküle, welche in der Lage sind, eine antiproliferative Aktivität auszuüben.
